# EUROPEAN PATENT APPLICATION

(11) **EP 4 623 961 A1**
(43) Date of publication of application: **01.10.2025**
(21) Application number: 24166326.9
(22) Date of filing: 26.03.2024
(51) Int. Cl.: A61M 5/315, A61M 5/20, A61M 5/24, A61M 5/32

(54) **INJECTION DEVICE WITH NEAR FIELD COMMUNICATION READER**

(71) Applicant: Ypsomed AG, 3401 Burgdorf (CH)
(72) Inventor: Schüpbach, Simon, 3400 Burgdorf (CH)
(74) Representative: Meier Obertüfer, Jürg

(57) **Abstract**

The present application relates to an injection device (1) comprising a housing (3) defining an inner space and a product container received into the inner space. The injection device further comprises a drive element which is configured to move a piston of the product container in the discharge direction, and an electronic element connected to the drive element. The housing of the injection device comprises at least one near field communication reader (4) connected to the electronic element. The electronic element is configured to read information from a near field communication transmitter device (8) and to perform specific actions depending on the information read from the near field communication transmitter device.

## Description

### Technical Field

The invention relates to an injection device with a near field communication reader which is able to perform specific actions based on information read by the near field communication reader from a near field communication transmitter device. Further, the invention also relates to an injection system with such an injection device as well as at least one near field communication transmitter device, especially a near field communication tag or a near field communication enabled mobile device.

### Background Art

A variety of diseases exist that require regular treatment by subcutaneous administration of a drug, and several drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container, wherein the drive spring may have to be charged or strained manually prior to injection of a dose. A manually powered delivery drive requires a user to manually provide the energy to move the piston, for instance by applying a distal force component to the injection device.

The drug dose to be injected may typically be manually selected by turning a dosage knob and observing the actual dialed dose from a dose window or display of the injection device. A dose is dispensed by inserting the needle into a suited portion of human skin and by moving the piston manually or by pressing a release button of an automatic injection device. Automatic injection devices may comprise an electronic dose dial mechanism to automatically set a dose.

Also known is the use of autoinjectors with syringes. Autoinjectors usually comprise a body for housing a syringe as well as a drive mechanism in order to move the plunger of the syringe upon actuation of the autoinjector. The drive mechanism typically comprises a drive means, such as an electric motor or a spring for moving a drive element, for example a rod, which acts on the piston of the syringe. The prefilled syringe comprises an injection needle or cannula that is permanently affixed to a first end of the syringe. The syringe is typically sealed at a second end thereof by the piston.

For safety and hygiene reasons, it is desirable that the injection needle does not protrude from a housing of the injection device except for the time when the injection needle is used for injection of a drug. Thus, either the needle is moved out of the housing for the injection and back into the housing after injection or the injection device includes a needle protection sleeve which may be moved to unsheathe the injection needle for the injection, and which may be moved back to a needle protection position after the injection.

Most injection devices are configured as single use devices which incorporate both the drug container and the drive mechanism in the same housing. Such devices are usually to be disposed after the injection for hygiene reasons.

Disposable injection devices comprising an electric actuator or an electronic control require a source of energy which is usually in the form of a battery. However, in this case, the injection devices may not be disposed in the regular waste but must be subjected to special disposal or to recycling, which is an additional burden to the patient. Further, disposing a battery, motor and/or electronics after a single use is a waste of resources and increases the costs of the therapy.

Hence, semi-reusable injection devices have been developed. Such semi-reusable injection devices typically comprise a reusable drive unit as well as a disposable syringe module which may be releasably coupled or attached to the drive unit. The drive unit usually includes the drive mechanism and electronics whereas the syringe module includes the syringe with the injection needle and a needle protection sleeve. The patient can thus discard the syringe module when it is empty or after use and can load the drive unit with a new syringe module for a next injection.

Injection devices usually comprise input means which allow a patient to trigger specific actions to be performed by the injection device. Such input means are ordinarily configured as push buttons, rotatable knobs or the like and hence rely on tactile interaction of the patient with the input means. However, such tactile interactions may occur accidentally, e.g. when the injection device is grasped or hold by a patient, thus leading to the triggering of unwanted actions. If such an unwanted action leads to the unsheathing of the injection needle, it may bare a risk of injury to the patient There is hence the need to provide injection devices which are safer to handle and which avoid the risk that unwanted actions are accidentally triggered by patients.

### Summary of the invention

It is thus the object of the invention to create a drug delivery device which is safer to use and which avoids the triggering of unwanted actions by patients.

The solution of the invention is specified by the features of claim 1. According to the invention the drug delivery device comprises a main housing and a releasably attachable product container holder The drug delivery device further comprises a drive element which is configured to move a piston of a product container held in the container holder in a discharge direction, and an electronic element connected to the drive element. The main housing of the drug delivery device comprises at least one near field communication reader connected to the electronic element. The near field communication reader is adapted to read information from a near field communication transmitter device and the electronic element is configured to perform specific actions depending on the information read from the near field communication transmitter device.

With a drug delivery device according to the present invention it is thus possible to trigger specific actions of the drug delivery device by using a near field communication transmitter device, such as a near field communication tag or a near field communication capable mobile device, for example a smartphone or tablet computer. Therefore, the drug delivery device according to the present invention may be designed devoid of any dedicated input means such as buttons or knobs relying on tactile interaction, thus ensuring that no unwanted actions are triggered when manually handling the injection device.

The term "injection device" or "injector" refers to a device that is removed from a puncture site after each product discharge. This in contrast to an "infusion system" or "infusion device" which comprises a cannula or needle that remains in the skin of a patient for a prolonged period of time, for example, several hours or even day.

The term "drug" as used herein comprises any flowable medical formulation suitable for controlled administration through a means such as a cannula or injection needle, for example comprising a liquid, a solution, a gel, or a fine suspension containing one or more active medical ingredients. A medical formulation may comprise a single active ingredient or be a mixed or co-formulated composition comprising a plurality of active ingredients. A medical formulation may comprise drugs such as peptides (e.g., insulins, insulin-containing preparations, GLP-1-containing, GLP-1-derived or GLP-1-analogous preparations), proteins, hormones, biologically obtained or artificial active ingredients, active ingredients based on hormones or genes, nutrient formulations, enzymes and further substances both in solid (suspended) or liquid form but also polysaccharides, vaccines, DNA, RNA, or other oligonucleotides, antibodies or parts of antibodies, and suitable bases, auxiliary and excipient substances.

The main housing preferably has an elongated shape. Preferably, the main housing is sleeve-shaped and/or cylindrical, in particular circular cylindrical. The main housing thereby includes or defines a longitudinal axis. Alternatively, the main housing may be of any other suitable shape, such as cuboid or any other polyhedron shape.

The main housing includes a distal end and a proximal end. As used herein, the distal end is the end of the main housing which faces skin of a patient during liquid product discharge, i.e. during injection, and the proximal end is the end of the main housing facing away from the skin of a patient during liquid product discharge. The main housing is preferably made of a polymer material. Use of a polymer material simplifies the production of the housing, as the housing may be injection molded.

The main housing encloses an inner space. At least the distal end of the main housing is preferably open, to allow the insertion of a product container holder containing a product container such as a prefilled syringe or a cartridge. The product container has a volume of between 0.1 ml and 3 ml, more preferably of 0.5 ml to 2.5 ml, and is made of glass or a polymer material. The prefilled syringe has a hollow injection needle or cannula permanently attached or staked to a barrel, and protected by a rigid needle shield, which in turn is removed from the needle together with a device cap. Alternatively, the container holder may comprise a mounting portion for mounting an injection needle thereto and establishing a fluid communication with the cartridge before an injection. The product container holder is configured to be inserted into the inner space of the main housing through a distal opening of the main housing and locked to the latter by appropriate locking means. Such a part re-usable injection device is for example described in EP 4 275 719 A1.

The container holder is preferably configured to hold the product container in such a manner that the product container cannot be displaced along the longitudinal axis of the housing, i.e. in an axially fixed manner. The product container holder in turn is preferably fixedly attached to an inner wall of the main housing. In this case, a tip of the injection needle protrudes out of the distal end of the main housing and the container holder. This allows the needle to be inserted into a puncture site of a patient by means of a movement of the housing towards the patient. Preferably, if the product container is arranged within the container holder in an axially fixed manner, a needle protection sleeve is provided, which radially encloses the needle and forms the distal end of the injection device when no injection is taking place. The needle protection sleeve is movable relative to the container holder in the direction of the longitudinal axis thereof. The needle protection sleeve preferably is arranged in an initial position such that the protection sleeve radially covers the injection needle. The needle protection sleeve is displaceable relative to the housing from its initial position in the proximal direction by an actuating stroke into an actuated position. In the actuated position the tip of the injection needle protrudes from a distal end or through an opening of the needle protection sleeve. Preferably, the needle protection sleeve may subsequently be displaced distally relative to the housing, e.g. by a needle protection stroke, from the actuated position into a needle protection position. In the needle protection position, the distal end of the needle protection sleeve is located distally relative to the tip of the injection needle tip, i.e. the needle protection sleeve completely covers the injection needle radially, in order to prevent a risk of injury from an exposed tip of the injection needle after the injection device has been used or after the product discharge.

The needle protection sleeve is preferably moved against the force of a at least one second spring, which can be referred to as needle shielding spring, in the proximal direction. The at least one second spring preferably moves the needle protection sleeve from the actuated position into the needle protection position. Preferably, the injection device comprises a locking member, which, for example, is resiliently arranged at the distal end of the housing and locks the needle protection sleeve in the needle protection position. This means that the locking member prevents the needle protection sleeve to be again moved in the proximal direction or to be moved from the needle protection position only to such an extent that the tip of the injection needle does not protrude from the distal end of the needle protection sleeve.

Alternatively, the product container may be displaceable arranged within the housing, i.e. such that it can be linearly displaced along the length axis of the housing and relative thereto, preferably at least in the direction of the distal end of the housing. This may be achieved either by configuring the container holder such as to be movable within the inner space of the housing or by providing means allowing the product container to be movable within the container holder. In this case, the injection needle is preferably directly coupled to a distal end of the product container, such that displacement of the product container in the distal direction relative to the housing allows a tip of the injection needle to emerge from an opening at the distal end of the housing, thus enabling for an automatic insertion of the needle tip into skin of a patient.

Preferably, the product container is configured as a syringe, which has a syringe body with the injection needle firmly attached to a first end thereof. The syringe body is preferably cylindrical and surrounds a volume, within which the piston is arranged. In the context of syringes, the piston is also usually referred to as plunger. The piston seals against the inside diameter of the syringe body and is displaceable with respect to the syringe body in the dispensing direction, i.e. towards the first end of the syringe body for product discharge. The liquid product is arranged between the piston and the injection needle prior to product discharge and preferably in the delivery state of the injection device. By the displacement of the piston relative to the syringe body in the dispensing direction, the liquid product is pushed out of the product container and into the injection needle.

The drive element is configured to be brought into an active connection with a piston of a product container held in the container holder. Preferably, the injection device comprises at least one component which exerts a force in the dispensing direction on the drive element. Preferably, the at least one actor includes a pre-tensioned first spring, such as for example a helical spring or a torsion spring. Alternatively, the at least one component may be a pre-tensioned elastic element, an electromechanical motor or any other suitable type. The at least one component is able to exert a force in the dispensing direction on the drive element. The drive element preferably is sleeve shaped and arranged within the inner space of the housing in a linearly displaceable manner, preferably in the direction of the longitudinal axis of the housing. The drive element preferably rests on the piston of the product container with a part thereof.

Hence, the drive element may be used to deliver a discharge stroke on the piston in order to push out the liquid product from the product container and through the injection needle. The drive element is preferably coupled to a trigger element, such as for example a button, located on an outside of the housing, which is actionable by a patient in order to trigger the discharge stroke.

Alternatively, the trigger is actionable by a movement of a needle protection sleeve from the initial position towards the actuated position, as described above.

The electronic element preferably comprises at least one integrated circuit or microcontroller which is able to process signals as well as a printed circuit board. The electronic element may further comprise an electric power source, such as a battery, in order to power the electronic element and the near field communication reader. The electronic element preferably comprises at least one non-volatile memory, such as to store data or program code to be executed when specific information is read by the near field communication reader. Further, the electronic element may also comprise a wireless communication module, such as e.g. a Bluetooth ^{®} module in order to transmit and/or receive data from an external device.

The near field communication reader may be arranged either within the inner space of the housing or on an outer surface of the housing. Near field communication (NFC) is a set of communication protocols that enables electronic devices to communicate with each other over a distance of approximately 4 cm or less. Near field communication relies on inductive coupling between electromagnetic coils present on near field communication enabled devices, and is hence based on radio-frequency identification (RFID) technology. Modern mobile phones or tablet computers are usually near field communication enabled and may be used as near field communication transmitter device according to the present invention. Alternatively or additionally, the near field communication device according to the present invention may be in the form of a near field communication tag. A near field communication tag is in principle a small memory element with near field communication capability.

It is to be noted that in the present invention, the term near field communication transmitter device encompasses both devices with an actively powered coil, such as e.g. a smartphone, as well as devices with a passively powered coil, i.e. which are powered through an electric current induced within the coil through an external magnetic field, such as the aforementioned near field communication tags. In the case of a near field communication tag, this induced electric current is used to retrieve information from the memory of the near field communication tag and to generate a magnetic field to transmit this information back to the actively powered device. Usually, near field communication tags include a capacitor which is charged by the induced electric current until a sufficient charge thereof is reached, upon which the information retrieval and information transmittal is initiated.

The near field communication reader of the injection device is connected to the electronic element and hence receives electric power from the electronic element in order to actively generate a magnetic field. In other words, the near field communication reader of the injection device acts as receiver of information stored in the near field communication transmitter device and the magnetic field generated by the near field communication reader may be used to induce electrical current in a near field communication transmitter device to power the latter.

The information which is read from the near field communication reader of the injection device preferably is a code which corresponds to the specific action which is to be performed by the injection device. Hence, the electronic element preferably comprises a look-up table stored in a non-volatile memory which enables the electronic element to perform the specific action associated with the code read from the near field communication transmitter device.

In principle, any action that may be performed by the injection device may be triggered by means of the near field communication transmitter device, such as, for example, display of battery status, display of information relating to the liquid product and/or product container, switching on and off of the injection device, transmission of data to a remote device, or unlocking of the product container holder from the main housing.

Therefore, the injection device is preferably used together with a set of near field communication transmitter devices configured as tags, each of the tags being associated to a specific action to be carried out by the injection device. Hence, a patient simply has to select the appropriate tag associated with a specific action the patient wants the injection device to execute, and to bring the tag into close proximity to the injection device.

Alternatively, a patient may also use a mobile device, such as a mobile phone or tablet computer in order to have the injection device perform a specific action. Thereby, the mobile device has to be near field communication capable and run an application which allows the patient to select the specific action to be performed by the injection device, whereupon the application generates and sends the corresponding information by means of near field communication to the injection device. Of course, as with a near field communication tag, the mobile device thereby has to be brought into close proximity to the injection device.

The near field communication reader continuously generates a near field communication electromagnetic field such that information from any near field communication transmitter device brought into proximity of the injection device may be read at any time. Preferably, however, the near field communication reader and the electronic element are switched off when the injection device is not used and only activated, i.e. switched on prior to the use of the injection device by a patient. In order to activate the electronic element and the near field communication reader the injection device may comprise an on/off-switch. Alternatively, the injection device may comprise other means of activation, such as for example a movement sensor, typically in the form of an accelerometer, which activates the electronic element and the near field communication reader when the injection is picked up by a patient. In a further alternative, in the case that the injection device is configured as re-usable injection device, the housing may comprise a switch or sensor which detects the insertion of a new product container or syringe module into the housing and subsequently activates the electronic module and the near field communication reader.

Preferably, the drug delivery device does not comprise any other means for control by a patient with the exception of the near field communication reader and concurrent control of the drug delivery device by movement of the device to or from the delivery site, in particular an injection trigger action by movement of a needle protection sleeve from the initial position towards the actuated position. This means that the housing of the injection device is preferably devoid of any push button, knob or other HMI means of dedicated tactile interaction. This greatly reduces the risk that a patient inadvertently triggers an unwanted action when handling the injection device. In other words, triggering an injection by retracting the needle protection sleeve is not considered a dedicated tactile interaction, and triggering the injection by the near field communication transmitter device is not considered practicable.

Preferably, the injection device comprises a non-tactile display, the electronic element being connected with the display and being configured to display a menu item of an array of menu items on the display, wherein the electronic element is further configured to display the next menu item in the array when a specific information is read from the near field communication transmitter device.

This enables a patient to switch or toggle through menu items by bringing a specific near field communication transmitter device in close proximity to the injection device. In order to switch through the array of menu items a patient simply has to repeatedly bring the specific near field communication transmitter device into close proximity with the injection device, whereupon a next menu item in the array is shown each time the near field communication transmitter device is brought into close proximity with the injection device.

The menu items may for example be the battery load status of the electronic element, serial number of the injection device, expiry date of the liquid product and the like.

In order to select the specific menu item, another near field communication transmitter device comprising information to cause the injection device to select this menu item is preferably brought into proximity of the injection device. In this embodiment, the electronic element is preferably configured to perform a specific action linked with the selected menu item or to display items of a sub-menu, which are preferably stored in a sub-menu array.

Preferably, the electronic element is configured to perform a specific action when the specific information is read from the near field communication transmitter device for a time which is longer than a defined time.

Hence, a patient may select to have an action corresponding to the menu item shown to be performed by the injection device simply by holding the near field communication transmitter device in close proximity to the injection device for a longer time.

Preferably, the defined time is longer than 3 seconds, more preferably longer than 5 seconds. Further preferably, the defined time is shorter than 10 seconds.

Preferably, the product container comprises a near field communication tag and the at least one near field communication reader is arranged in or on the housing such that it is able to read information from the near field communication tag of the product container as well as information form the near field communication transmitter device.

Therefore, the electronic element may read information from the near field communication tag of the product container, which may contain e.g. information on the liquid product contained in the product container, a batch number of the liquid container, an expiration date of the liquid product or the like. In this embodiment, the electronic element is preferably configured to be able to show at least part of the information read from the near field communication tag of the liquid container, e.g. on a display of the injection device. Further, the electronic element is preferably configured to block the drive element from moving in the dispensing direction depending on the information read from the near field communication tag of the liquid container, e.g. when liquid product is expired.

Preferably, the main housing and the product container holder are adapted such that the product container holder is removable from the main housing and a new product container holder is attachable to the main housing.

Preferably, the injection device comprises at least one sensor to detect the insertion of a new product container, wherein the at least one sensor is connected to the electronic element and the electronic element is configured to read information from the near field communication tag of the new product container when the insertion of the new product container is detected by the at least one sensor.

Therefore, the electronic element may read information specific for the new product container inserted into the inner space, such as e.g. information relating to the composition of the liquid product, the expiration date of the liquid product, a batch number of the liquid product, the volume of the product container and the like.

Preferably, the electronic element is configured not to perform any action when the piston is moved in the discharge direction by the drive element. This prevents that any action to be performed by the injection system interferes with product discharge, hence ensuring that the entire dose of the liquid product is delivered to the patient.

The present invention further relates to a drug delivery system comprising a drug delivery device according to the description above and at least one near field communication transmitter device comprising information to cause the drug delivery device to perform at least one specific action.

Preferably, the near field communication transmitter device is a near field communication tag or a near field communication enabled mobile device, preferably a mobile phone.

In the case that the at least one near field communication transmitter device is a near field communication tag, the injection system preferably comprises a plurality of near field communication tags, each near field communication tag comprising information to cause the injection device to perform a different specific action. Preferably, each near field communication tag includes a readable label, a pictogram and/or a colour which enables a patient to recognize which near field communication tag causes the injection device to perform which specific action.

If the at least one near field communication transmitter device is a mobile device, the mobile device preferably includes an application, i.e. a software program product, which enables a patient to select a specific action to be performed by the injection device, whereupon the application sends corresponding information via a near field communication module of the mobile device to the injection device. The application preferably is stored in a memory of the mobile device. Preferably, the application is provided as downloadable file which may be installed on a mobile device of a patient.

Other advantageous embodiments and combinations of features come out from the detailed description below and the entirety of the claims.

### Brief description of the drawings

The drawings used to explain the embodiments show:
- Fig. 1: a perspective view of an embodiment of an injection device according to the present invention;
- Fig. 2: a perspective detailed view of the syringe module according to the embodiment shown in Fig. 1;
- Fig. 3: an explosion view of the syringe module according to Fig. 2;
- Fig. 4: a sectional view of the housing according to the embodiment as shown in Fig. 1 without the syringe module;
- Figs. 5: a schematic representation of the working principle of an injection device well as an injection system according to the present invention.

In the figures, the same components are given the same reference symbols.

### Preferred embodiments

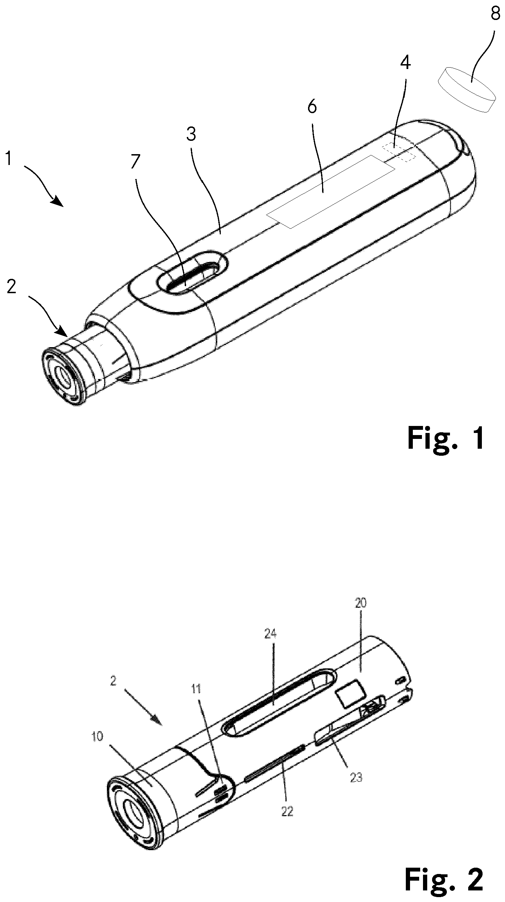

Fig. 1 shows a perspective view of an embodiment of an injection device 1 according to the present invention. The injection device 1 comprises a housing 3 which defines an inner space into which a syringe module 2 including a product container holder holding a syringe with an attached injection needle is inserted from a distal end. In the figure, the distal end of the injection device is on the left while the proximal end thereof is on the right. The syringe module 2 is inserted into the inner space of the housing 2 through an opening provided at the distal end of the housing. A near field communication reader 4 is provided inside the inner space of the housing 3 in the embodiment shown. The injection device 1 further comprises a display 6 on which information can be shown as well as a window opening 7 through which the product container may be viewed, such that a patient may visually confirm that the product container is indeed still full prior to product discharge, i.e. prior to an injection.

The near field communication reader 4 is connected to an electronic element inside the inner space of the housing 3 and is able to read information from a near field transmitter device 8, which is shown as a near field communication tag in the embodiment shown. The electronic element is configured to perform a specific action according to the information read from the near field communication transmitter device 8 by the near field communication reader 4, such as e.g. display a next menu item from an array of menu items stored in a memory of the electronic element, indicate a battery status by lighting an LED, display information relating to the composition of the liquid product, the expiration date of the liquid product, a batch number of the liquid product, the volume of the product container, transmission of data to a remote device, or unlocking of the syringe module from the main housing.

The combination of injection device 1 and near field communication transmitter device 8 corresponds to an embodiment of an injection system according to the present application.

Fig. 2 shows a perspective detailed view of the syringe module 2 according to the embodiment shown in Fig. 1. The syringe module 2 has an elliptical form in the cross section and extends along a longitudinal axis. On a distal end a cap 10 is mounted in an initial or delivery state. The proximal end includes a holding structure to releasably attach the syringe module 2 to the inner space of the housing 3.

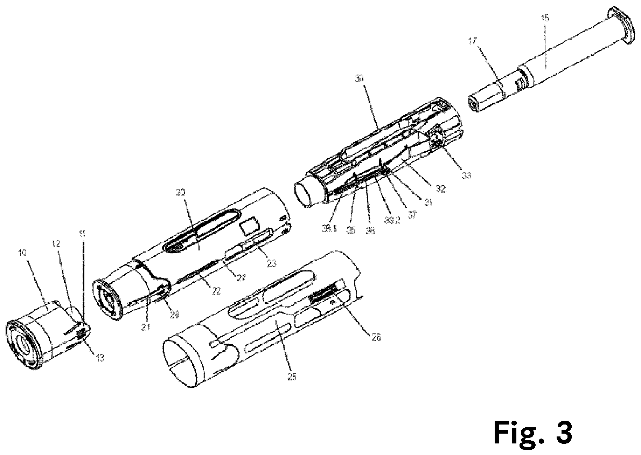

Fig. 3 shows an explosion view of the syringe module 2 according to Fig. 2. As shown in Fig. 3 the syringe module 2 includes a product container holder 30, a needle protection sleeve or cover sleeve 20 coaxially arranged around the product container holder 30 and movable relative thereto along the longitudinal axis and a near field communication tag 25 wrapped around the cover sleeve 20. Information provided in the near field communication tag 25 may also be read by the near field communication reader 4 once the syringe module 2 is inserted into the inner space of the housing 2. A prefilled product container 15 comprising a liquid product filled therein and a rigid needle shield 17 (RNS) initially mounted on the injection needle are arranged in the product container holder 30. The product container holder 30, the cover sleeve 20 and the cap 10 are individually injection-molded plastic parts which are all made of the same material, preferably polypropylene.

As shown in Fig. 3 the cover sleeve 20 has a non-circular cross section and in particular an elliptical form in a plane perpendicular to the longitudinal axis. The cover sleeve comprises a window 24 allowing a patient to view the fill level of the product container 15 inside the syringe module 2. The window 24 is thereby arranged such as to be congruent with the window opening 7 of the housing once the syringe module is arranged in the inner space of the housing 2.

The near field communication tag 25 is wrapped around an outer surface of the syringe module 2. The near field communication tag 25 comprises a small memory unit (not shown) and a coil 26. The memory includes information about the syringe module 2, e.g. for identification thereof, information about the composition of the liquid product inside the product container 15, the volume of the liquid product contained in the product container 15, expiry date and dispensing parameter such as injection speed and holding time or the like.

The product container 15 includes the syringe barrel and a piston 16 movably arranged therein. The injection needle 18 is non-releasably connected to a distal end of the syringe barrel. In an unused state the injection needle 18 is covered by the rigid needle seal 17. The latter in turn is axially and radially fixedly connected to the cap 10 by the holder 12 once the product container 15 is mounted to the product container holder 30.

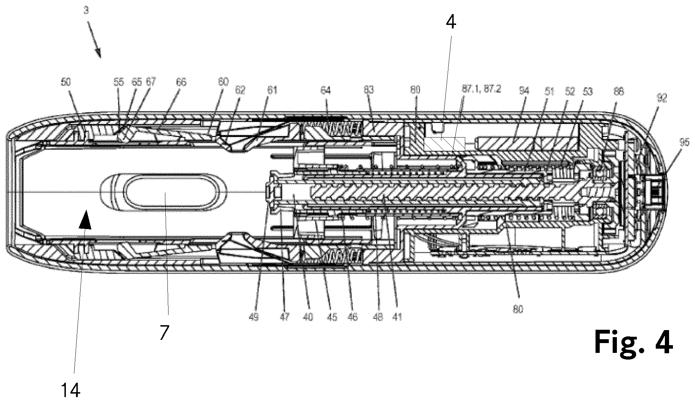

Fig. 4 shows a sectional view of the housing 3 according to the embodiment as shown in Fig. 1 without the syringe module 2. The cut of the sectional view runs along the longitudinal axis of the housing 3. The housing 3 includes an opening on its distal end adapted to accommodate a proximal portion of the syringe module 2. A support structure 80 accommodates the electronic element 92 and the near field communication reader 4. The electronic element 92 is in electrical communication with the near field communication reader 4, the display 6 and the battery 94. By means of the battery 94, the electronic element may supply the display 6 as well as the near field communication reader 4 with electrical power. Information read by the near field communication reader 4 are relied to the electronic element 92 by means of the cables, whereupon the electronic element 92 performs a specific action according to the information read by the near field communication reader 4, e.g. such as to display specific data on the display 6.

When the syringe module 2 is inserted as described, the proximal end of the syringe module 2 moves a detection pin 97 proximally and thus a switch 96.1 of a position sensor automatically sends a signal to the electronic element 92 which detects the presence of the syringe module 2 inside the housing 3. The electronic element 92 is thus activated. The near field communication reader 4 is in sequence powered on and reads information from the near field communication tag 25 provided on the syringe module 2. Further, the near field communication reader 4 now emits a near field communication signal such as to be able to read information from a near field communication transmitter device 8 which is brought into proximity of the injection device 1, as exemplarily shown in Fig. 1.

The information read from the near field communication tag 25 of the syringe module may be validated by the electronic element 92, e.g. by comparing the expiry date of the syringe module 2 with the current date or by checking whether a syringe module 2 with the correct liquid product has been inserted.

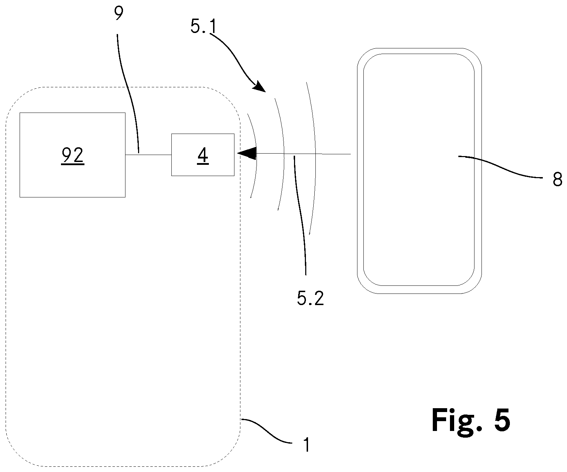

Fig. 5 shows schematically the working principle of an injection device 1 (only represented schematically with dashed lines) well as an injection system according to the present invention. The near field communication reader 4 is connected to the electronic element 92 by means of cables 9, which provide the near field communication reader 4 with electric power (the electronic element 92 being itself connected to the battery 94, see e.g. Fig. 5a). Further, the cables 9 also provide for information transfer between the near field communication reader 4 and the electronic element 92. The near field communication reader 4 emits an electromagnetic field 5.1 at a frequency of 13.56 MHz by means of a coil (not shown) provided therein. In the event that a near field communication transmitter 8, which is represented as a mobile phone in the embodiment shown is brought into proximity of the injection device 1, the electromagnetic field 5.1 triggers a reply 5.2 from the near field communication transmitter device 8. With said reply 5.2, the near field communication transmitter device 8 transmits information to the near field communication reader 4. The information is received, i.e. read by the near field communication reader 4 and relied to the electronic element 92. Typically, the information read from the near field communication transmitter device 8 by the near field communication reader 4 is in the form of a specific code. The electronic element 92 then causes the injection device 1 to perform a specific action according to the information provided by the near field communication transmitter device 8. For example, the electronic element 92 may compare the information provided with a look-up table which associates specific codes to specific actions to be performed by the injection device 1. In the case that the near field communication transmitter device 8 is in the form of a near field communication tag which itself does not comprise any source of electric power, the magnetic field 5.1 emitted from the near field communication reader 4 may induce a current in a coil provided in the near field communication tag which serves to power a reply from the near field communication tag, i.e. the transmission of information from the near field communication tag to the near field communication reader 4.

## Claims

1. A drug delivery device (1) for dispensing a liquid product, comprising a main housing (3) containing a drive element, an electronic element connected to the drive element, as well as a product container holder (30) releasably attachable to the main housing and holding a product container (16) comprising a piston therein, the piston being movable in a dispensing direction in order to discharge the liquid product from the product container, wherein the drive element is adapted to move the piston in the discharge direction, **characterized in that** the main housing comprises a near field communication reader (4) connected to the electronic element, wherein the electronic element is adapted to receive information from a near field communication transmitter device (8) read by the near field communication reader, and to perform specific actions depending on the information from the near field communication transmitter device.

2. The drug delivery device according to claim 1, **characterized in that** the electronic element is exclusively controlled by the information from the near field communication transmitter device and/or by movement of a needle protection sleeve which radially encloses, in a non-actuated position, a needle in fluid communication with the product container.

3. The drug delivery device according to any of claims 1 or 2, **characterized in that** the injection device comprises a display, the electronic element being connected to the display and being configured to cause the display to show a menu item from an array of menu items, wherein the electronic element is further configured to display the next menu item in the array when a specific information is read from the near field communication transmitter device.

4. The drug delivery device according to any of claims 1 to 3, **characterized in that** the electronic element is configured to perform a specific action when the specific information is read from the near field communication transmitter device for a time which is longer than a defined time.

5. The drug delivery device according to any of claims 1 to 4, **characterized in that** the product container or the product container holder comprises a near field communication tag and **in that** the near field communication reader is arranged in or on the housing such that it is able to read information from the near field communication tag as well as information form the near field communication transmitter device.

6. The drug delivery device according to any of claims 1 to 5, **characterized in that** the main housing and the product container holder are adapted such that the product container holder is removable from the main housing and a new product container holder is attachable to the main housing.

7. The drug delivery device according to claim 5 and 6, **characterized in that** the drug delivery device comprises a sensor to detect attachment of a product container holder to the main housing, wherein the sensor is connected to the electronic element and wherein information from the near field communication tag of the product container or the product container holder is read when the attachment of the product container holder is detected by the sensor.

8. The drug delivery device according to any of claims 1 to 7, **characterized in that** the electronic element is configured to not perform any action while the piston is being moved in the discharge direction by the drive element.

9. A drug delivery system comprising a drug delivery device according to any of claims 1 to 8 and at least one near field communication transmitter device comprising information to cause the drug delivery device to perform at least one specific action.

10. The drug delivery system according to claim 9, **characterized in that** the near field communication transmitter device is a near field communication tag or a near field communication enabled mobile device, preferably a mobile phone.
